# EUROPEAN PATENT APPLICATION

(11) **EP 4 674 942 A1**
(43) Date of publication of application: **07.01.2026**
(21) Application number: 23925328.9
(22) Date of filing: 02.03.2023
(51) Int. Cl.: C12M 1/34

(54) **INSPECTION DEVICE FOR FOREIGN MATTER AND PARTICLE AGGREGATES, AND INSPECTION METHOD**

(71) Applicant: HITACHI HIGH-TECH CORPORATION, Tokyo 105-6409 (JP)
(72) Inventor: SUGIYAMA, Kiyotaka, Tokyo 105-6409 (JP)
(74) Representative: MERH-IP Matias Erny Reichl Hoffmann Patentanwälte PartG mbB
(86) International application number: PCT/JP2023/007891
(87) International publication number: WO 2024/180774

(57) **Abstract**

Provided is an inspection device that can detect foreign matter and fine particle aggregates, and can accurately detect the degree of proliferation of fine particles. An inspection device 500 for aggregates comprises an image acquisition unit 11 that acquires an image of the inside of a container 511 holding a liquid containing fine particles, and detects the presence or absence of a fine particle aggregate. The inspection device 500 comprises: a foreign matter presence/absence determination unit 24 that binarizes an image, creates a first binarized image, and determines the presence or absence of foreign matter; a fine particle region detection unit 25 that extracts contours of the image to produce a contour-extracted image, binarizes a composite image of the extracted contour-extracted image and the image, creates a second binarized image, and detects a fine particle region; an integrated signal difference processing unit 16 that calculates the difference value between a first integrated signal, which was obtained by integrating the first binarized image, and a second integrated signal, which was obtained by integrating the second binarized image; and an aggregation presence/absence determination unit 17 that determines the presence or absence of aggregates by numerically analyzing the difference waveform of the difference value. The inspection device detects the presence or absence of at least two different types of objects contained in the image.

## Description

### Technical Field

The present disclosure relates to an inspection device and an inspection method for detecting foreign matter and fine particle aggregates and accurately detecting the degree of proliferation of fine particles.

### Background Art

International epidemics of infectious diseases are a major concern for human health worldwide. In recent years, various emerging infectious diseases have been spreading one after another, and in response, new inspection methods for specifying and detecting causative viruses and causative bacteria, and new vaccines have developed.

Here, bacterial infection will be exemplified. Currently, the emergence of multidrug-resistant bacteria has become a serious problem on a global scale, and the US CDC states that carbapenem-resistant Acinetobacter, carbapenem-resistant Enterobacteriaceae, Clostridiodis difficile, and the fungus Candida auris are major threats.

If an outbreak of such resistant bacteria occurs, the risk of death for patients is high. It is important to promptly identify the causative bacteria and measure susceptibility thereof to detect drug-resistant bacteria and promptly perform appropriate treatment. In an identification test (ID) for identifying the causative bacteria, a method using mass spectrometry is mainly used, and it is now possible to perform tests within one hour from colony or positive blood culture samples.

In a susceptibility testing (AST) for evaluating susceptibility of bacteria to drugs, a test using turbidity measurement is mainly used, but to speed up the test, a method for capturing images of bacteria with a microscope and detecting a degree of proliferation of the bacteria with a sensitivity higher than that in a method in the related art has been put into practical use.

In the AST using images, there is a method in which fluorescently labeled bacteria are mixed with an antibacterial agent and cultured, and images of the bacteria are captured with a fluorescent microscope, and the degree of proliferation is detected from changes in the fluorescent image, thereby determining the susceptibility. In addition, there is a method in which image processing is performed on a bacterial image acquired by a bright-field microscope without using a fluorescent label, and a change in the shape or a degree of proliferation of bacteria is detected, thereby determining the susceptibility. The latter technique using a bright-field microscope is a label-free detection method, and therefore has the advantage of being able to reduce reagent costs.

However, in the case of the label-free detection method, there is a disadvantage that objects other than bacteria, such as dust, dirt, and air bubbles contained in samples or test containers, are misdetected as bacteria. For example, if dust, dirt, and air bubbles other than bacteria are erroneously regarded as bacteria, it may be erroneously determined that the bacteria have multiplied even when they have not.

Here, when considering objects that are contained in samples used in the susceptibility testing and may appear in images, there are the test target bacteria, the cells in a biological sample from which the bacteria are isolated, such as red blood cells, white blood cells, and platelets, as well as other particles such as dust, dirt, and air bubbles. In addition, some species of bacteria may produce viscous substances such as mucoid through their own metabolism, or exhibit coagulation properties through actions of extracellular enzymes such as coagulase, causing the bacteria to clump together and these clumps may appear in the images.

Since incident light is scattered and absorbed in a region in which dust, dirt, and air bubbles are present, a pixel luminance value of this region decreases and appears dark. Similarly, when bacteria aggregate, the pixel luminance value of the aggregated region decreases, and the region appears dark in the image, making it difficult to distinguish between the two. Meanwhile, the inside of aggregated bacteria is less susceptible to the action of an external antibacterial agent and may exhibit resistance, making it an important indicator in the test. If dust, dirt, air bubbles, and aggregated bacteria can be accurately distinguished and detected, an effective indicator indicating proliferation in a short-time culture may be obtained.

PTL 1 discloses a method for correctly detecting a region of fine particles corresponding to bacteria by performing a logical product operation of images before and after contour extraction and then performing binarization to prevent erroneous recognition.

PTL 2 discloses a method for extracting noise from an appearance frequency of a shape feature of an individual cell.

### Citation List

### Patent Literature

PTL 1: JP7130109B
PTL 2: JP2011-243188A

### Summary of Invention

### Technical Problem

In the technique disclosed in PTL 1, both a region of fine particles that appear isolated and a region of fine particles that appear densely and dark are detected. Therefore, although the detection sensitivity of fine particles is high, it is difficult to distinguish and detect isolated bacteria, aggregated bacteria, and foreign matters other than the bacteria, such as dust, dirt, and air bubbles.

In the technique disclosed in PTL 2, since the noise extraction is performed from information such as a size of an object, it is difficult to distinguish and detect a foreign matter and a bacteria aggregate because differentiation cannot be made from shape information.

Therefore, in an image including a measurement target having a particle shape such as proliferating bacteria, a method for distinguishing and detecting bacteria, a bacteria aggregate, and foreign matters other than bacteria, such as dust, dirt, and air bubbles, while maintaining the sensitivity of bacteria detection has not been disclosed so far.

An object of the present disclosure is to provide an inspection device and an inspection method capable of detecting the foreign matter and the fine particle aggregate and accurately detecting the degree of proliferation of fine particles.

### Solution to Problem

To achieve the above object, the present disclosure is formed as follows.

An inspection device for an aggregate for detecting presence or absence of a fine particle aggregate includes: an image acquisition unit configured to acquire an image of an inside of a container that holds a liquid containing a fine particle; a foreign matter presence or absence determination unit configured to binarize the image, create a first binarized image, and determine presence or absence of a foreign matter; a fine particle region detection unit configured to extract a contour of the image to obtain a contour-extracted image, binarize a composite image of the extracted contour-extracted image and the image, create a second binarized image, and detect a fine particle region; an integrated signal difference processing unit configured to calculate a difference value between a first integrated signal obtained by integrating the first binarized image and a second integrated signal obtained by integrating the second binarized image; and an aggregation presence or absence determination unit configured to determine presence or absence of an aggregate by numerically analyzing a difference waveform of the difference value, in which presence or absence of at least two different types of objects included in the image is detected.

An inspection method for an aggregate for acquiring an image of an inside of a container that holds a liquid containing a fine particle to detect presence or absence of a fine particle aggregate includes: binarizing the image, creating a first binarized image, and determining presence or absence of a foreign matter; extracting a contour of the image to obtain a contour-extracted image, binarizing a composite image of the extracted contour-extracted image and the image, creating a second binarized image, and detecting a fine particle region; calculating a difference value between a first integrated signal obtained by integrating the first binarized image and a second integrated signal obtained by integrating the second binarized image; and determining presence or absence of an aggregate by numerically analyzing a difference waveform of the difference value, in which presence or absence of at least two different types of objects included in the image is detected.

### Advantageous Effects of Invention

According to the present disclosure, an inspection device and an inspection method capable of detecting the foreign matter and the fine particle aggregate and accurately detecting the degree of proliferation of fine particles can be provided.

### Brief Description of Drawings

[FIG. 1A] FIG. 1A is a diagram illustrating an example of an image including an air bubble as an example of a foreign matter.
[FIG. 1B] FIG. 1B is a diagram illustrating an example of an image including bacteria.
[FIG. 1C] FIG. 1C is a diagram illustrating an example of an image including bacteria obtained by proliferating and aggregating bacteria in a certain limited region.
[FIG. 2] FIG. 2 is a flowchart illustrating details of image processing according to Embodiment 1.
[FIG. 3A] FIG. 3A is a graph illustrating a result when a circular foreign matter considered to be an air bubble is included in the image.
[FIG. 3B] FIG. 3B is a diagram illustrating a result when no foreign matter is contained and uniformly dispersed bacteria are contained.
[FIG. 3C] FIG. 3C is a diagram illustrating a result when aggregated bacteria are contained although no foreign matter is contained.
[FIG. 4] FIG. 4 is a diagram illustrating a method for correcting a signal.
[FIG. 5] FIG. 5 is a diagram illustrating a schematic configuration example of a bacteria inspection device according to Embodiment 1.
[FIG. 6] FIG. 6 is a functional block diagram of a determination processing unit included in a control unit according to Embodiment 1.
[FIG. 7] FIG. 7 is an operation flowchart of determination of a bacterial degree of proliferation according to Embodiment 2.
[FIG. 8] FIG. 8 is a diagram illustrating culture time dependence of an area value of a bacterial region as an example of a feature calculated by removing influences of the foreign matter.
[FIG. 9] FIG. 9 is a functional block diagram of a determination processing unit included in a control unit according to Embodiment 2.
[FIG. 10] FIG. 10 is a float chart of image-capturing and image processing according to Embodiment 3.
[FIG. 11] FIG. 11 is a diagram illustrating a configuration example of a GUI that outputs a result.
[FIG. 12] FIG. 12 is a functional block diagram of a determination processing unit included in a control unit according to Embodiment 3.

### Description of Embodiments

According to the present embodiment, for example, a technique is disclosed in which a region of a fine particle is detected in an image of fine particles captured by a microscope in a well that holds a liquid containing fine particles while preventing erroneous recognition, a region where the fine particles aggregate and a region where other foreign matters are present are detected, the presence or absence and sizes of the aggregate and the foreign matter are determined, a feature of the fine particle is corrected by using information about a region of the foreign matter to accurately determine a degree of proliferation of the fine particle based on the feature.

Hereinafter, embodiments of the present disclosure will be described with reference to the drawings. Although the accompanying drawings illustrate specific embodiments based on principles of the present technique, the embodiments are provided for easily understanding the present technique and are not to be used to limit the interpretation of the present disclosure. In all the embodiments and all the drawings for illustrating the embodiments, components having the same functions may be denoted by the same reference numerals, and repeated description thereof may be omitted.

Examples of fine particle include bacteria, archaea, fungi, and biological cells, and bacteria will be described here as one type of the fine particle.

### Embodiments

### <Description of Image>

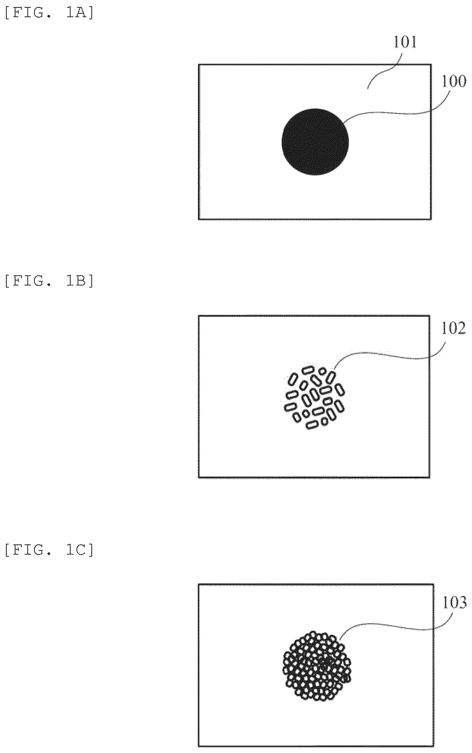

FIG. 1A, FIG. 1B, and FIG. 1C are diagrams illustrating examples of images including bacteria as fine particles in a sample solution or foreign matters other than bacteria. Here, for example, a grayscale image with 256 gradations in 8 bits will be described.

FIG. 1A is a diagram illustrating an example of an image including an air bubble as an example of a foreign matter 100. A region where the foreign matter 100 is present has a reduced amount of light received by an image-capturing unit due to absorption, refraction, or scattering, has a luminance less than that in a region of a background 101, and appears dark in the image.

FIG. 1B is a diagram illustrating an example of an image including bacteria 102. The bacteria 102 appears in a circular shape or an elliptical shape in the image, and when a focus is appropriately focused and an image is captured, a difference in shading occurs between a contour of the bacteria 102 and a central portion of the bacteria 102. For example, a luminance value of a contour portion of the bacteria 102 is less than a luminance value of the background 101, and a luminance value of the central portion of the bacteria 102 is larger than the luminance value of the background 101.

In general, since the bacteria 102 can freely move in a sample liquid, the bacteria 102 are often present in a uniformly dispersed manner. However, the bacteria 102 may be collected in one place depending on a sample state or an external environment, and a state of being collected in a circular shape as illustrated in FIG. 1B is considered. When the proliferation of the bacteria does not sufficiently progress, the region of the background 101 is present in a region of the bacteria 102, and therefore, both are spatially distinguished from each other.

FIG. 1C is a diagram illustrating an example of an image including bacteria 103 obtained by proliferating and aggregating bacteria in a certain limited region. When the bacteria 103 are densely packed, since a contour of the bacteria 103 is common to a contour of the adjacent bacteria 103, a clear difference in shading cannot be obtained as in FIG. 1B, and a region with a larger luminance value and a region with a less luminance value than that of the background are locally present.

Here, in the method disclosed in PTL 1, both the bacteria 102 and the aggregated bacteria 103 can be detected. Meanwhile, a region of the foreign matter 100 is also detected. In general, since a diameter of bacteria is about 0.5 µm to several µm and a size of an air bubble as the foreign matter 100 is several tens of µm or more, the bacteria 102 and the foreign matter 100 can be distinguished from each other by the size.

However, a size of the aggregated bacteria 103 is as width wide as several µm to several hundred µm, and it is difficult to distinguish the aggregated bacteria 103 and the foreign matter 100 from each other in consideration of dirt that can have various sizes as the foreign matter. In addition, even when attention is paid to shape information, it is difficult to distinguish the aggregated bacteria 103 because the aggregated bacteria 103 are collected in any shape.

In view of the above circumstances, a method for distinguishing and detecting aggregated bacteria and a foreign matter and determining the presence or absence of an aggregate and the presence or absence of the foreign matter will be described.

### (Embodiment 1)

### <Details of Image Processing Method according to Present Disclosure>

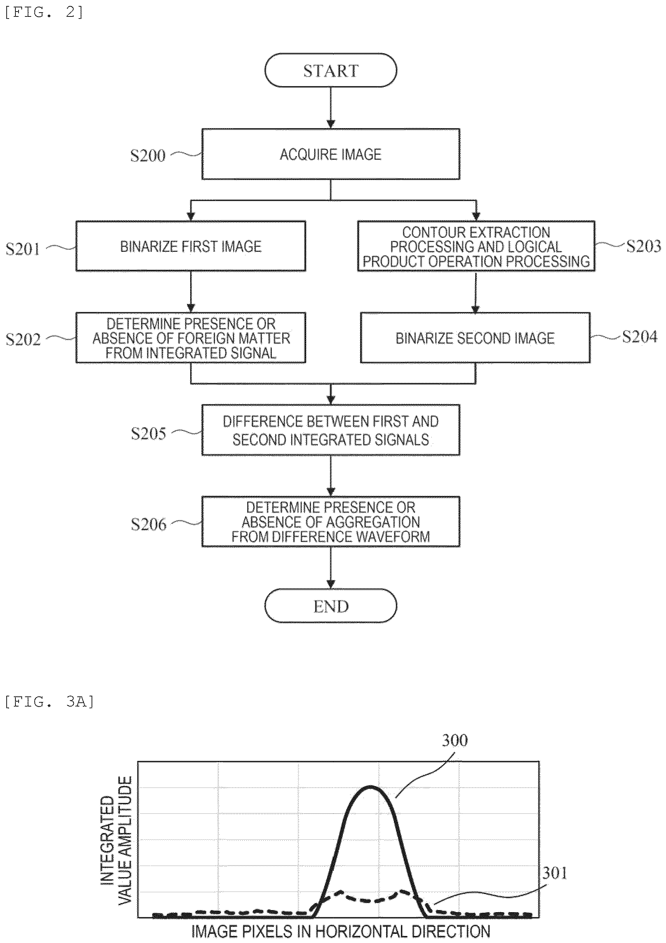

FIG. 2 is a flowchart illustrating details of image processing.

### (1) Step S200

In step S200, an image including bacteria is acquired. In Embodiment 1, the image is an 8-bit grayscale image, with a pixel value of 0 being black and a pixel value of 255 being white. The processing according to the present embodiment is also effective for a grayscale image other than 8 bits or an image with black and white reversed. The same processing is also possible for a color image by converting the color image into color spaces such as R, G, and B or H, S, and V.

### (2) Step S201

In step S201, binarization processing is performed on a first image acquired in step S200. In the binarization processing, to detect a foreign matter that appears dark, it is preferable to perform binarization using a luminance value less than that of the background as a binarization threshold. To detect a foreign matter that appears brighter than the background, a luminance value larger than that of the background may be used as the binarization threshold.

The threshold may be a preset fixed value, or may be dynamically determined for each image by calculating the inter-class separation in a histogram, for example, as in Otsu's method. Alternatively, for example, when the luminance is not uniform in an image plane due to variation in the amount of illumination light, the binarization threshold may be dynamically changed for each small region of the image.

### (3) Step S202

In step S202, pixels detected in the binarization processing in step S201 are integrated in at least one of vertical or horizontal direction (a first binarized image is created). A luminance value of the binarized image is 0 or 255, and for example, when a foreign matter that appears dark is detected and pixels are integrated in the vertical direction, the image is scanned in the vertical direction, a pixel having a luminance value of 255 is ignored, and a pixel having a luminance value of 0 is sequentially added with 1 to obtain an integrated signal in the vertical direction.

For example, when assuming that an image has vertical 100 pixels × horizontal 200 pixels, it is possible to obtain a vertical direction integrated signal having a width 100 × a maximum amplitude 200 and a horizontal direction integrated signal having a width 200 × a maximum amplitude 100.

When a foreign matter is present in the image, a fluctuation peak occurs in the integrated signal. Therefore, the foreign matter can be detected by numerically analyzing the integrated signal. Specifically, first, smoothing is performed to remove noise in the integrated signal. For example, it is preferable to execute moving average or lowpass filter processing. Thereafter, it is detected whether there is a fluctuation peak. Simply speaking, a standard deviation or a fluctuation coefficient of the signal is calculated, and it is determined whether a value thereof exceeds a reference value.

More specifically, a first derivative and a second derivative of the signal may be calculated, an extreme value of the signal may be determined, an amplitude of a peak may be calculated, and it may be determined whether a value thereof exceeds the reference value. When a fluctuation peak is present, it is determined that a foreign matter is present.

The detection of the fluctuation peak described above may be performed on only one of the integrated signals in the vertical and horizontal directions, or may be performed on both of them. In a more preferable method for detecting a foreign matter with high sensitivity, a fluctuation peak detection is performed for both integrated signals in the vertical and horizontal directions, and it is determined that a foreign matter is present when the fluctuation peak is detected in any one of the signals.

In addition to the method described above, the presence or absence of a foreign matter may be determined by recognizing a lump of particles from the binarized image generated in step S201 and comparing a size or shape information of the recognized particles, for example, circularity, roundness, aspect ratio, and perimeter with a threshold. In this method, since the calculation load increases when many particles are found, the detection method for numerically analyzing the integrated signal is preferable from the viewpoint of high-speed processing.

### (4) Step S203

In step S203, the first image acquired in step S200 is copied to a memory, and contour extraction processing is executed. For example, a Sobel filter or a Laplacian filter that extracts edges (contour), or a variance filter that replaces an own luminance value with a variance value of a luminance of the surrounding N × N pixels, may be executed. Thereafter, a logical calculation, specifically, a logical product operation is performed on a luminance value of each pixel of the first image copied to the memory and an image after the edge extraction (a contour-extracted image) to acquire a second image (a composite image (a second binarized image) of the extracted contour and the first image).

### (5) Step S204

In step S204, binarization processing is performed on the second image acquired in step S203. The binarization processing is performed in the same manner as in step S201. By performing the binarization processing on the second image, an image in which all regions of bacteria, aggregated bacteria, and foreign matters are detected is obtained.

### (6) Step S205

In step S205, a first integrated signal of a binarized image of the first image and a second integrated signal of a binarized image of the second image are calculated, and a difference value between the first integrated signal and the second integrated signal is calculated. Since the integrated signal of the binarized image of the first image is acquired in step S202, the integrated signal of the binarized image of the second image is calculated in step S205, and the difference value between the two integrated signals is calculated. In the present processing, the difference value between the integrated signals may be calculated in any one of the vertical and horizontal directions of the image, but more preferably, the difference values between the integrated signals in both directions are calculated.

### (7) Step S206

In step S206, the presence or absence of an aggregate is determined by numerically analyzing the difference value of the integrated signals acquired in step S205. Since a region of the foreign matter is detected in the first image and regions of bacteria, aggregated bacteria, and foreign matter are detected in the second image, it is determined that the aggregated bacteria are present when there is a fluctuation peak in the difference value of the integrated signals. The detection of the fluctuation peak is performed in the same manner as in step S202, and a method for comparing a deviation value after smoothing with a threshold or detecting an extreme value from the first derivative and the second derivative of the signal and comparing the extreme value with the threshold is preferable.

Here, since the calculation of the difference value in step S205 and step S206 is to extract the difference between two signals, it is not necessary to calculate the difference value, and the same effect can be obtained by calculating a ratio of the two signals.

### <Effects Related to Detection of Foreign Matter and Aggregates in Present Disclosure>

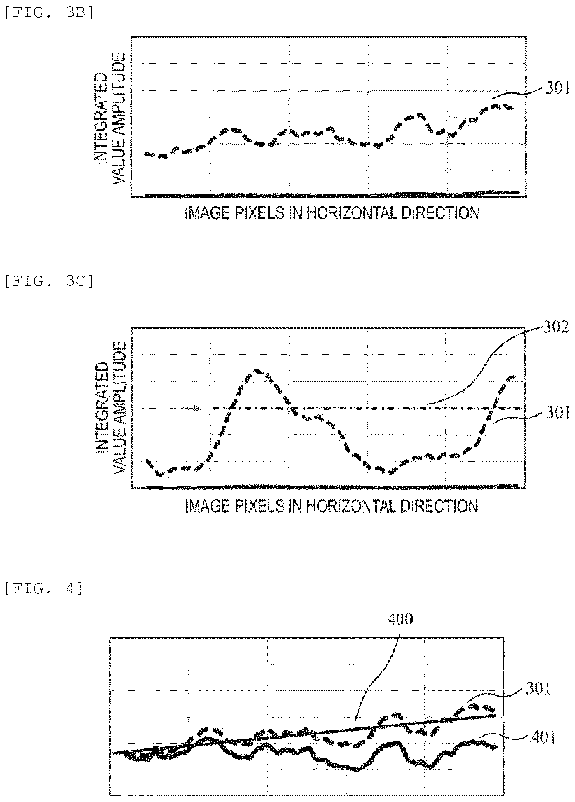

FIG. 3A, FIG. 3B, and FIG. 3C illustrate waveforms of integrated signals calculated according to the workflow described above.

FIG. 3A illustrates a result when a circular foreign matter considered to be an air bubble is included in the image. Here, for example, when integration is performed in the vertical direction of the image, an integrated signal 300 of the binarized first image is indicated by a solid line, and a difference signal 301 of the integrated signal is indicated by a broken line. When a foreign matter is included, only the integrated signal 300 of the binarized first image has a large fluctuation peak.

FIG. 3B illustrates results when no foreign matter is included and uniformly dispersed bacteria are included. In this case, no clear peak is present in any signal.

FIG. 3C illustrates a result when no foreign matter is included and aggregated bacteria are included. In this case, only the difference signal 301 of the integrated signal has a large fluctuation peak.

By numerically analyzing the signal described above, the presence or absence of the foreign matter and the aggregated bacteria is determined, and meanwhile, the difference signal 301 of the integrated signal in FIG. 3B is inclined, and it is preferable to perform the analysis after correcting the waveform.

FIG. 4 illustrates a method for correcting a signal.

First, an approximate straight line 400 is calculated for the difference signal 301. For example, it is preferable to use the least squares method for the approximate straight line 400. A corrected difference signal 401 can be obtained by calculating a slope of the approximate straight line 400 and subtracting a value of the slope. After such correction, a standard deviation of the waveform is calculated as follows.

Regarding the determination of the foreign matter, the standard deviation of the integrated signal after binarization of the first image in FIG. 3A in which the foreign matter is present is 166, the standard deviation of the integrated signal after binarization of the first image in FIG. 3B in which the foreign matter is not present is 4, and the presence or absence of the foreign matter can be detected by comparing the values of the standard deviation with, for example, a threshold (80).

Regarding the determination of the aggregate, the standard deviation of the difference signal of the integrated signal in FIG. 3A in which the aggregate is not present is 30, the standard deviation of the difference signal of the integrated signal in FIG. 3B is 27, and the standard deviation of the difference signal of the integrated signal in FIG. 3C in which the aggregate is present is 121. In this case, similarly, the presence or absence of the aggregate can be detected by comparing the values of the standard deviation with, for example, a determination threshold (80).

Although not illustrated, it is possible to exclude the influence of bacteria that are uniformly present in the space by calculating an average value and subtracting the average value from the corrected difference signal 401, and it is possible to more accurately detect the aggregate.

As described above, it is possible to detect the presence or absence of foreign matter and aggregates by performing correction processing on the integrated signal, calculating the standard deviation of the waveform as waveform and numerical analysis, and comparing the standard deviation with the determination threshold.

Although the present method shows simplified results, similar results can be obtained by implementing the method to detect the presence or absence of foreign matter or aggregates by calculating the derivatives of the signal, detecting a peak, and comparing the peak with the determination threshold, as described in step 202. In addition, although the results are not shown, it is possible to detect peaks of both the foreign matter and the aggregated bacteria, and it is also possible to determine an image in which the foreign matter and the aggregated bacteria are simultaneously included.

### <Configuration Example of Bacteria Inspection Device>

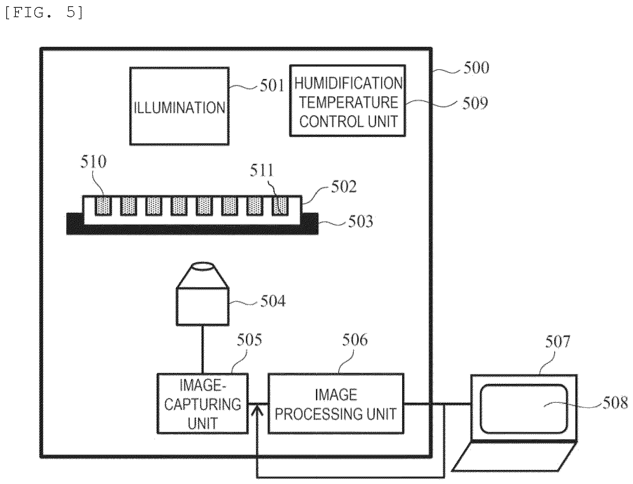

FIG. 5 is a diagram illustrating a schematic configuration example of a bacteria inspection device (aggregate inspection device) 500 according to Embodiment 1 of the present disclosure. The bacteria inspection device 500 includes an illumination unit 501, an inspection plate 502, a stage 503, an objective lens 504, an image-capturing unit 505, an image processing unit 506, and a control unit 507.

The inspection plate 502 includes one or more containers (wells) 511, preferably 96 wells or 384 wells, and a sample solution 510 is held in each container (well). The sample solution 510 contains a bacterial sample, and may also contain a medium for bacterial growth or one or more antibacterial agents having a pre-adjusted concentration.

Since the inspection plate 502 changes depending on the type of bacteria as the inspection target, the type of drug, and the like, it is preferable that a plurality of the inspection plates 502 different for each inspection are introduced into the bacteria inspection device 500. The inspection plate 502 is preferably distinguished by a number for distinguishing an ID of a patient who receives an inspection, the type of bacteria as the inspection target, and the type of drug, and is preferably identified by, for example, a barcode label, a two-dimensional code, or RFID.

The illumination unit 501 emits light in a direction of the inspection plate 502. The illumination unit 501 may use a light source such as a white light source such as a lamp, or an LED that includes light in a specific wavelength region. The light that has passed through each well and the sample solution 510 in the inspection plate 502 is condensed by the objective lens 504 and formed into an image and captured by the image-capturing unit 505. Then, for example, by moving the stage 503 under the control of the control unit 507 and changing a relative position between the well of the inspection plate 502 and the image-capturing unit 505, images of different wells can be captured. The image-capturing operation is also controlled by the control unit 507 and is executed at preset time intervals, for example, every 30 minutes. The image acquired by the image-capturing unit 505 is processed by the image processing unit 506 and transmitted to the control unit 507.

Here, a focal point of the objective lens 504 is preferably aligned with a well bottom surface of the inspection plate 502, and may be aligned with an inside of the sample solution 510 separated from the bottom surface. Images may be captured at a plurality of locations in the well, or a plurality of images of the inside of the sample solution 510 separated from the well bottom surface of the inspection plate 502 may be captured.

The control unit 507 is, for example, a general computer, and includes an input and output unit 508 in addition to controlling the image-capturing unit 505 and the image processing unit 506. The input and output unit 508 is a display, a mouse, a keyboard, or a touch panel, and displays measurement conditions and outputs of the inspection.

It is preferable that the bacteria inspection device 500 includes a humidification temperature control unit 509 and the entire image-capturing system for acquiring images is controlled to, for example, about 35°C and 30% in humidity, because the bacterial growth efficiently proceeds.

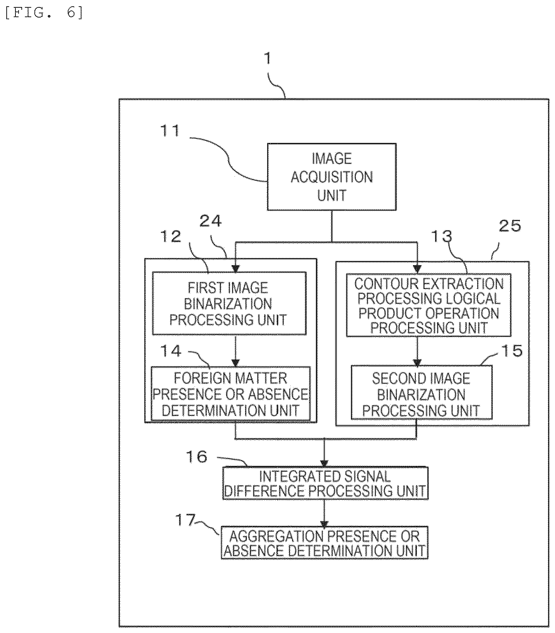

FIG. 6 is a functional block diagram of a determination processing unit 1 included in the control unit 507. The determination processing unit 1 executes an operation flow in FIG. 2.

In FIG. 6, the determination processing unit 1 includes an image acquisition unit 11, a first image binarization processing unit 12, a contour extraction processing logical product operation processing unit 13, a foreign matter presence or absence determination unit 14, a second image binarization processing unit 15, an integrated signal difference processing unit 16, and an aggregation presence or absence determination unit 17.

The first image binarization processing unit 12 and the foreign matter presence or absence determination unit 14 implement a foreign matter presence or absence determination unit 24. The contour extraction processing logical product operation processing unit 13 and the second image binarization processing unit 15 calibrate a fine particle region detection unit 25.

The image acquisition unit 11 executes the processing in step S200 in FIG. 2, and the first image binarization processing unit 12 executes the processing in step S201. The contour extraction processing logical product operation processing unit 13 executes the processing in step S203, and the foreign matter presence or absence determination unit 14 executes the processing in step S202. The second image binarization processing unit 15 executes the processing in step S204, and the integrated signal difference processing unit 16 executes the processing in step S205. The aggregation presence or absence determination unit 17 executes the processing in step S206.

Since Embodiment 1 has the configuration described above, it is possible to provide an inspection device and an inspection method capable of detecting the foreign matter and the fine particle aggregate and accurately detecting the degree of proliferation of fine particles.

### (Embodiment 2)

Next, Embodiment 2 will be described.

Embodiment 2 is an example in which Embodiment 1 is applied, an influence of the foreign matter is removed, a feature of bacteria included in the image is calculated, and a degree of proliferation of the bacteria is determined.

Since an overall configuration of a bacteria inspection device according to Embodiment 2 is the same as the configuration illustrated in FIG. 5, illustration and detailed description thereof will be omitted.

### <Detection of Foreign Matter and Aggregates and Determination of Bacterial Degree of Proliferation from Feature>

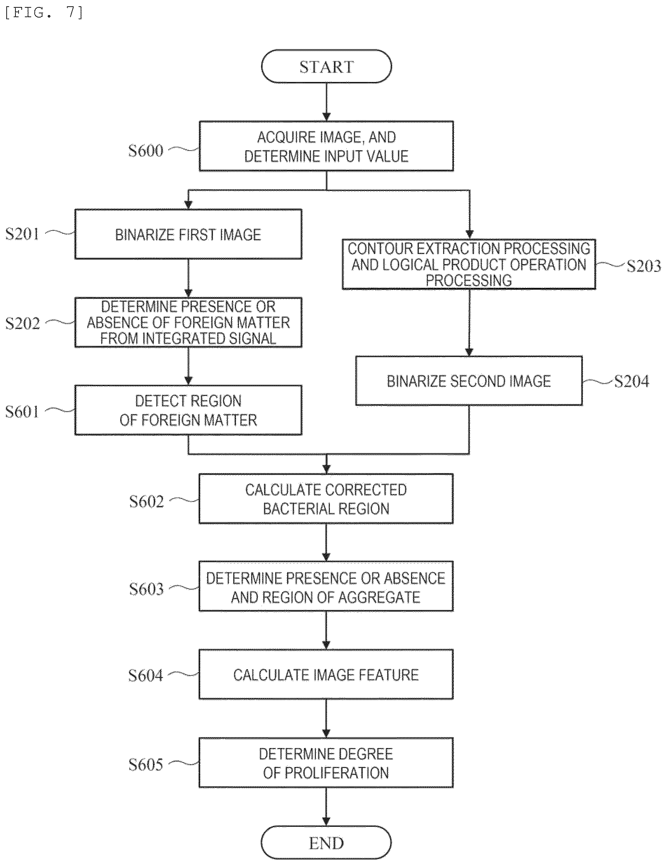

FIG. 7 is an operation flowchart of the determination of the bacterial degree of proliferation according to Embodiment 2. The determination of the bacterial degree of proliferation will be described with reference to FIG. 7.

### (1) Step S600

In step S600, the image processing unit 506 receives the image acquired by the image-capturing unit 505, stores the image in an internal memory (not illustrated) of the image processing unit 506 or a memory (not illustrated) of the control unit 507, and executes processing of subsequent steps. Here, a luminance value of the input image is preferably determined in advance (an input value is determined). For example, when an abnormality in the illumination unit 501 or an abnormality in a positional relationship between the inspection plate 502 and the stage 503 is predicted, such as when the luminance values of all the pixels are 0 or 255, it is preferable to determine an error without performing subsequent processing.

In addition, when the culture time has progressed sufficiently and the bacteria have proliferated excessively, the entire image may be filled with the aggregated bacteria, making it difficult to distinguish between the foreign matter and the aggregate. In this case, it is possible to determine the state since an average value or a standard deviation of the histogram of the luminance values of the image is abnormally low or high, and for example, only step S203 and step S204 may be executed, and step S201 to step S603 may not be executed.

### (2) Step S201, Step S202, Step S203, and Step S204

Step S201, step S202, step S203, and step S204 are the same as step S201, step S202, step S203, and step S204 illustrated in FIG. 2, and the details thereof will be omitted.

### (3) Step S601

Step S601 is executed only when the foreign matter is detected in step S202, and a foreign matter region in the image is detected.

For example, by using the binarized image acquired in step S201, one connected region can be regarded as the foreign matter region. The binarization processing may be newly performed on the image acquired in step S200. In the binarization processing, to detect a foreign matter that appears dark, it is preferable to perform binarization using a luminance value less than that of the background as a binarization threshold, to detect a foreign matter that appears brighter than the background, a luminance value larger than that of the background may be used as a binarization threshold, and a result obtained by superimposing both may be used. The threshold may be a preset fixed value, or may be dynamically determined for each image by calculating the inter-class separation in a histogram, for example, as in Otsu's method. Alternatively, for example, when the luminance is not uniform in an image plane due to variation in the amount of illumination light, the binarization threshold may be dynamically changed for each small region of the image.

To distinguish the aggregated bacteria from the foreign matter, additional processing may be performed on the binarized image. For example, in the aggregated bacteria 103 as illustrated in FIG. 1C, since a region with a larger luminance value and a region with a less luminance value than that of the background are locally present, binarization using a certain threshold may make it difficult to distinguish between aggregate and foreign matter regions. In this case, for example, by repeatedly performing erosion or dilation utilizing the Minkowski sum and Minkowski difference, the aggregated bacteria region is deleted, and only the foreign matter region remains, which is effective.

### (4) Step S602

In step S602, the foreign matter region is corrected to calculate a bacterial (fine particle) region. Specifically, the region after binarization calculated in step S204 includes both the bacterial region and the foreign matter region, and by performing difference processing on the foreign matter region calculated in step S601, the foreign matter region can be excluded, and the bacterial region can be calculated.

### (5) Step S603

In step S603, step S205 and step S206 illustrated in FIG. 2 are executed to determine the presence or absence of the aggregate, and then the size and the number of aggregates are calculated when the aggregate is present. For example, as illustrated in FIG. 3C, a determination threshold 302 is set in advance. If it is determined in step S206 that the aggregate is present, the integrated signal is compared with the determination threshold 302, and a region in which the integrated signal exceeds the determination threshold is set as an aggregate region. In the example of FIG. 3C, in a region of 630 to 1030 pixels and a region of 2300 to 2450 pixels in the horizontal direction, the integrated signal exceeds the determination threshold, and by approximating the aggregates as being circular, it is determined that aggregates having a diameter of 400 pixels and a diameter of 150 pixels are present.

FIG. 3C illustrates a result of integrating the difference waveform in the vertical direction of the image, and by performing the integration also in the horizontal direction and performing comparison with the determination threshold in the same manner, two-dimensional positions and size information about the aggregates can be calculated from the information about the aggregate in the vertical direction and horizontal direction in which calculation is performed. Although the processing time increases, the number and area of aggregates can be calculated more accurately without approximating the aggregates as being circular.

### (6) Step S604

In step S604, a feature is calculated from the corrected bacterial region calculated in step S602. Specifically, the number of small regions determined as bacteria, the average area or the total area of each small region, and numerical values indicating the shape of each small region, such as circularity, roundness, aspect ratio, perimeter, and degree of convexity, are calculated. It is preferable to average the numerical value indicating the shape of each small region, and it is preferable to output values obtained by averaging the numerical value by the number of small regions detected as bacteria.

Since the foreign matter region is corrected in step S602, an influence of the foreign matter is removed from the feature calculated in step S604.

For example, when the number of small regions determined as bacteria and a total area of small regions are calculated, simply subtracting only the foreign matter region does not take into account an influence of bacteria that are actually present in the foreign matter region, resulting in an underestimation of the value. In this case, a value obtained by estimating the influence of bacteria included in the foreign matter region can be calculated by performing the calculation of the feature after the difference × (area of the entire image)/{(area of the entire image) - (area of foreign matter)}.

In general, the image acquisition in step S600 is performed a plurality of times with the lapse of culture time. Therefore, steps from step S600 to step S604 may be performed only once, or steps from step S600 to step S604 may be repeatedly performed on a plurality of images captured in a certain cycle to calculate an image feature according to the number of images. The image feature may be calculated for an image obtained by capturing images of antibacterial agents of different concentrations or a sample containing antibacterial agents of different concentrations.

### (7) Step S605

In step S605, the degree of proliferation of bacteria is determined based on information about the image feature calculated in step S604. In the simplest method, the number of small regions determined as bacteria or the total area value of small regions can be treated as a factor indicating bacterial proliferation. Therefore, it is possible to determine whether there is proliferation by comparing the feature with a preset threshold.

It is also possible to determine the degree of proliferation of the fine particles by calculating either a total area value of the fine particles or the total number of the fine particles and eithr the number of aggregates or an area value of the aggregates as the feature of the fine particles.

Alternatively, it may be possible to determine whether there is proliferation by creating a discriminant analysis model based on separately prepared data and inputting the image feature to the model as an input. In the determination of whether there is proliferation, the number of aggregates and the total area of the aggregates calculated in step S603 may be used, and more accurate determination can be performed by combining the numerical information calculated in step S603 and the numerical information calculated in step S604.

In general, when a region occupied by the aggregates increases in an image, bacteria tend to proliferate, and thus this feature is information indicating proliferation. Since the agglutination ability of bacteria varies depending on the bacterial species and strain, more preferable results can be obtained by changing the determination threshold and the discriminant analysis model for each bacterial species and strain.

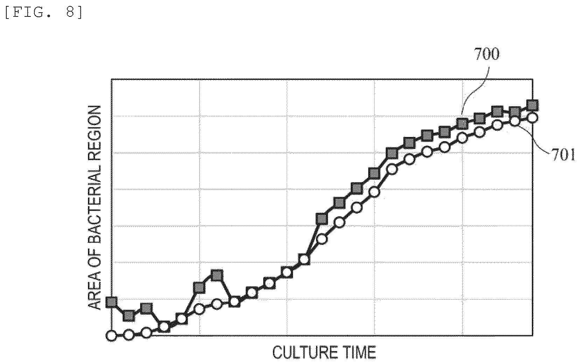

FIG. 8 is a diagram illustrating culture time dependence of an area value of the bacterial region as an example of the feature calculated by removing influences of the foreign matter. In FIG. 8, a vertical axis represents an area of the bacterial region, and a horizontal axis represents a culture time.

The culture time dependence of the area value of the bacterial region is calculated in the processing in step S604. For example, when a shadow caused by the foreign matter is present and a region in which the shadow appears changes from moment to moment, the feature repeatedly increases and decreases as in a feature 700 before correction. This increase or decrease is caused by the foreign matter, and may affect correct determination of the degree of proliferation. A feature 701 after correction indicates a monotonic increase, and the feature excluding the influence of the foreign matter can be calculated.

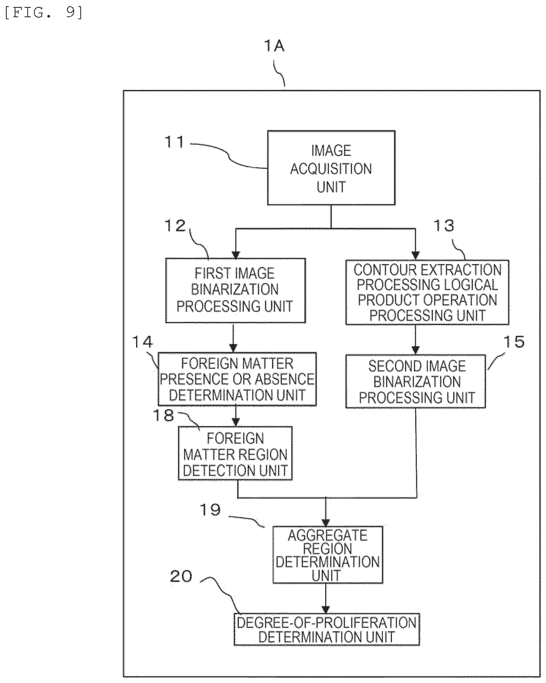

FIG. 9 is a functional block diagram of a determination processing unit 1A included in the control unit 507. The determination processing unit 1A executes the operation flow in FIG. 7.

In FIG. 9, the determination processing unit 1A includes the image acquisition unit 11, the first image binarization processing unit 12, the contour extraction processing logical product operation processing unit 13, the foreign matter presence or absence determination unit 14, a foreign matter region detection unit 18, the second image binarization processing unit 15, an aggregate region determination unit 19, and a degree-of-proliferation determination unit 19.

The image acquisition unit 11 executes the processing of step S600 in FIG. 7, and the first image binarization processing unit 12 executes the processing of step S201. The contour extraction processing logical product operation processing unit 13 executes the processing in step S203, the foreign matter presence or absence determination unit 14 executes the processing in step S202, and the foreign matter region detection unit 18 executes the processing in step S601. The second image binarization processing unit 15 executes the processing in step S204, and the aggregate region determination unit 19 executes the processing in step S602, step S603, and step S604. Then, the degree-of-proliferation determination unit 20 executes the processing in step S605.

Since Embodiment 2 has the configuration described above, similarly to Embodiment 1, it is possible to provide an inspection device and an inspection method capable of detecting the foreign matter and the fine particle aggregate and accurately detecting the degree of proliferation of fine particles.

### (Embodiment 3)

Next, Embodiment 3 will be described.

Embodiment 3 is an example in which Embodiment 1 and Embodiment 2 are applied, an image not including a foreign matter is acquired, and a feature of bacteria is calculated. Since an overall configuration of a bacteria inspection device according to Embodiment 3 is the same as the configuration illustrated in FIG. 5, illustration and detailed description thereof will be omitted.

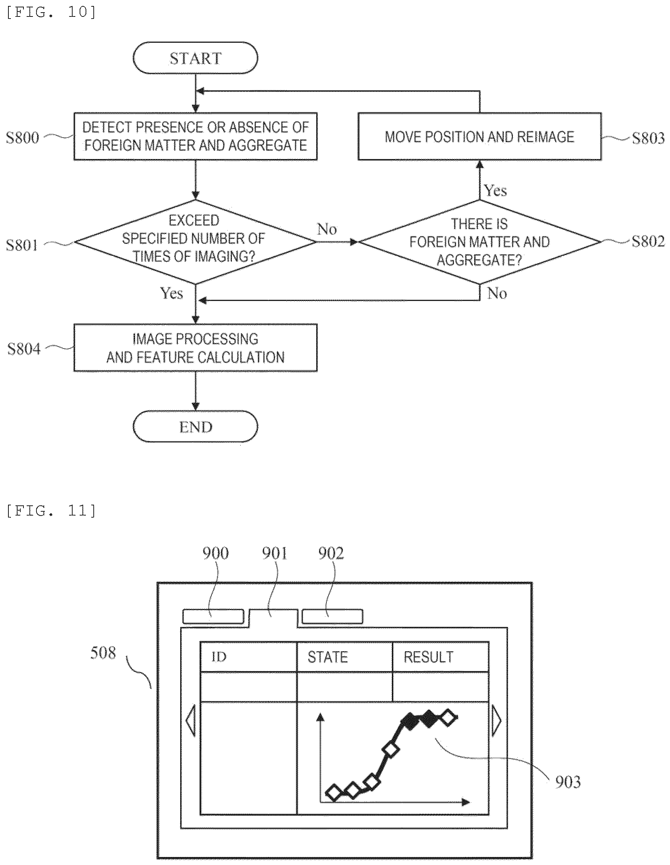

FIG. 10 is a float chart of imaging and image processing according to Embodiment 3.

### (1) Step S800

In step S800, presence or absence of foreign matter and aggregates is detected according to the float chart illustrated in FIG. 2.

### (2) Step S801

In step S801, the current number of times of imaging is compared with a predetermined threshold, and if the number of times is equal to or larger than the threshold, the processing proceeds to step 804. If the number of times is less than the threshold, the processing proceeds to step S802. For example, the number of times of imaging is preferably two or three. It is also possible to select an appropriate number of times of imaging according to the number of wells.

### (3) Step S802

In step S802, it is determined whether a foreign matter and an aggregate are detected in step 800. If neither is found, the processing proceeds to step S804. If a foreign matter is found, the processing proceeds to step S803. When an aggregate is found, the processing proceeds to step S803, but when it is desired to actively detect an aggregate, the processing may proceed to step S804. For example, when the bacteria serving as an analysis target are bacteria that easily aggregate and an aggregation state is desired to be detected with higher sensitivity, the processing may proceed to step S803 only when a foreign matter is found.

### (4) Step S803

In step S803, relative positions of the inspection plate 502, the objective lens 504, and the image-capturing unit 505 are changed, and imaging is performed again. Since an approximate value of a position where the foreign matter or the aggregate is present can be calculated from the results of the numerical analysis in step 202 and step 206, it is preferable to perform imaging again by moving, for example, the inspection plate 502 in a direction in which the foreign matter or the aggregate is predicted to be not present.

### (5) Step S804

In step S804, the feature is calculated according to the float chart in FIG. 7. For example, if it is determined in step S800 that there is no foreign matter or aggregate, there is no problem in performing the processing by skipping step S201, step S202, and step S601 to step S603 in FIG. 7.

### <Example of Result Output>

An example in which an image processing result of the present disclosure is transmitted to a user through the input and output unit 508 will be described.

FIG. 11 is a diagram illustrating a configuration example of a graphical user interface (GUI) that outputs results. In FIG. 11, the GUI includes, for example, a measurement condition display unit 900, a measurement result display unit 901, and a device state display unit 902. These display units are separately displayed on the monitor, and it is preferable that the display units can be switched by performing input from, for example, tabs indicating the display units or buttons indicating switching.

In the measurement condition display unit 900, conditions for performing bacterial inspection are displayed, and input and output are possible. For example, it is possible to display the measurement conditions of the inspection related to bacterial species serving as an inspection target, drugs, an inspection time, an imaging cycle of the images, and the like, and to receive outputs from the user.

In the device state display unit 902, information indicating a device state is displayed, and input and output are possible. For example, it is possible to display information about errors or warnings of the device, information about temperature and humidity, opening and closing information about a device door, information about device maintenance, and various types of setting information about the device, and to receive outputs from the user.

Results of the image processing calculated in the present disclosure are output to the measurement result display unit 901. For example, an ID of a specimen serving as the inspection target, a current measurement state of the specimen, a measurement result, and an image processing output result 903 are displayed. In a column of the measurement state of the specimen, it is preferable to display the measurement time up to the present, the time remaining until the measurement is completed, and an instruction to the user to take out the plate.

In a column of the measurement result, it is preferable to display the presence or absence of an error and a warning in the middle of measurement, and error information determined by the image processing, for example, information such as the presence or absence of a foreign matter or an aggregate. It is also preferable to have a plot of the time-dependent change in numerical information calculated by the image processing, which is useful for intuitively showing the degree of bacterial growth to the user. Specifically, each rectangular point in this plot represents a point where measurement is performed, and if there is a foreign matter or aggregates, a state may be clearly indicated, such as by being shown in a different color or shape from the others, and it is preferable that details of an error and the corresponding image can be output to the user by selecting the corresponding plot.

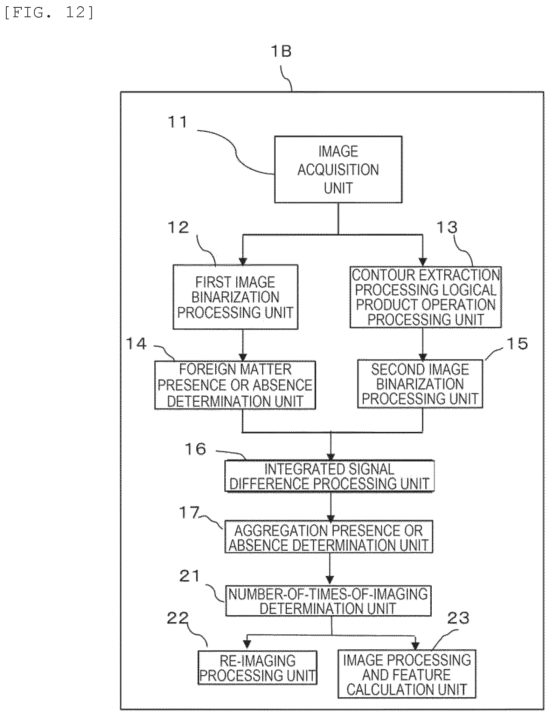

FIG. 12 is a functional block diagram of a determination processing unit 1B included in the control unit 507. The determination processing unit 1B executes the operation flow in FIG. 10. The determination processing unit 1B includes a number-of-times-of-imaging determination unit 21, a re-imaging processing unit 22, and an image processing feature calculation unit 23 in addition to the functional blocks illustrated in FIG. 6. The number-of-times-of-imaging determination unit 21 performs the operation processing in step S801. The re-imaging processing unit 22 performs step S802 and step S803, and the image processing feature calculation unit 23 performs the operation processing in step S804.

Since Embodiment 3 has the configuration described above, similarly to Embodiment 1, it is possible to provide an inspection device and an inspection method capable of detecting the foreign matter and the fine particle aggregate and accurately detecting the degree of proliferation of fine particles.

Functions of the embodiments described above can also be implemented by software program codes. In this case, a storage medium in which program codes are recorded is provided to a system or an apparatus, and a computer (or CPU or MPU) of the system or the apparatus reads the program codes stored in the storage medium.

In this case, the program codes read from the storage medium implement the functions of the embodiments described above, and the program codes and the storage medium in which the program codes are stored implement the present disclosure. Examples of the storage medium for supplying such program codes include a flexible disk, a CD-ROM, a DVD-ROM, a hard disk, an optical disk, a magneto-optical disk, a CD-R, a magnetic tape, a non-volatile memory card, and a ROM.

An operating system (OS) or the like operating on a computer may perform a part or all of actual processing based on an instruction of the program code, and the functions of the embodiments described above may be implemented by the processing. After the program codes read from the storage medium are written in a memory on the computer, a CPU or the like of the computer may perform a part or all of actual processing based on an instruction of the program code, and the function of the embodiment described above may be implemented by the processing.

The software program codes for implementing the functions of the embodiments may be stored, by distributing via a network, in a storage unit such as a hard disk or a memory of the system or the device or a storage medium such as a CD-RW or a CD-R, and the computer (or CPU or MPU) of the system or the device may read and execute the program code stored in the storage unit or the storage medium at the time of use.

Finally, it is necessary to understand that processes and techniques described here are not essentially related to any specific device, and can be implemented by any appropriate combination of components. Various types of devices for general purposes can be used in accordance with teachings described here. It may be advantageous to construct a dedicated device to execute steps of the method described here. Various inventions can be formed by appropriately combining a plurality of components disclosed in the embodiments. For example, several components may be deleted from all components disclosed in the embodiments. Further, components belonging to different embodiments may be appropriately combined.

Although the present disclosure has been described in relation to specific examples, the specific examples are used for description only, and are not intended to limit the present disclosure in all viewpoints. It is understood that there are many combinations of hardware, software, and firmware suitable for implementing the present disclosure for those skilled in the present field. For example, the above-described software can be implemented in a wide range of programs or script languages such as assembler, C/C++, perl, Shell, PHP, and Java (registered trademark).

Control lines and information lines considered to be necessary for the description are shown in the embodiments described above, and not all control lines and information lines in a product are necessarily shown. All configurations may be connected to one another.

The present disclosure is not limited to the embodiments described above and includes various modifications. For example, the embodiments described above are described in detail to describe the present disclosure in an easy-to-understand manner, and are not necessarily limited to including all the described configurations. A part of a configuration of a certain embodiment can be replaced with a configuration of another embodiment, and a configuration of another embodiment can be added to a configuration of a certain embodiment. A part of a configuration of each embodiment may be added to, deleted from, or replaced with another configuration.

The present disclosure includes the following inspection method for an aggregate.
(1) An inspection method for an aggregate for acquiring an image of an inside of a container that holds a liquid containing a fine particle to detect presence or absence of a fine particle aggregate, the inspection device including: a step of binarizing the image, creating a first binarized image, integrating the first binarized image at least in a vertical or horizontal direction, numerically analyzing an integrated value, and detecting a foreign matter to determine presence or absence of the foreign matter; and a step of changing a relative position between the container and the image-capturing unit and acquiringan image of the inside of the container again when it is determined that the foreign matter is present.
(2) The inspection method for an aggregate according to (1), further includes: a step of extracting a contour of the image to obtain a contour-extracted image, binarizing a composite image of the extracted contour-extracted image and the image, creating a second binarized image, and detecting a fine particle region; a step of calculating a difference value between a first integrated signal obtained by integrating the first binarized image and a second integrated signal obtained by integrating the second binarized image; and a step of determining presence or absence of an aggregate by numerically analyzing a difference waveform of the difference value.

### Reference Signs List

1, 1A, 1B: determination processing unit
11: image acquisition unit
12: first image binarization processing unit
13: contour extraction processing logical product operation processing unit
14: foreign matter presence or absence determination unit
15: second image binarization processing unit
16: integrated signal difference processing unit
17: aggregation presence or absence determination unit
18: foreign matter region detection unit
19: aggregate region determination unit
20: degree-of-proliferation determination unit
21: number-of-times-of-imaging determination unit
22: re-imaging processing unit
23: image processing feature calculation unit
24: foreign matter presence or absence determination unit
25: fine particle region detection unit
100: foreign matter
101: background
102: bacteria
103: aggregated bacteria
300: integrated signal of binarized first image
301: difference signal of integrated signal
302: determination threshold
400: approximate straight line
401: corrected difference signal
500: bacteria inspection device
501: illumination unit
502: inspection plate
503: stage
504: objective lens
505: image-capturing unit
506: image processing unit
507: control unit
508: input and output unit
509: humidification temperature control unit
510: sample solution
511: container (well)
700: feature before correction
701 feature after correction
900: measurement condition display unit
901: measurement result display unit
902: device state display unit
903: image processing output result

## Claims

1. An inspection device for an aggregate for detecting presence or absence of a fine particle aggregate, the inspection device comprising:
an image acquisition unit configured to acquire an image of an inside of a container that holds a liquid containing a fine particle;
a foreign matter presence or absence determination unit configured to binarize the image, create a first binarized image, and determine presence or absence of a foreign matter;
a fine particle region detection unit configured to extract a contour of the image to obtain a contour-extracted image, binarize a composite image of the extracted contour-extracted image and the image, create a second binarized image, and detect a fine particle region;
an integrated signal difference processing unit configured to calculate a difference value between a first integrated signal obtained by integrating the first binarized image and a second integrated signal obtained by integrating the second binarized image; and
an aggregation presence or absence determination unit configured to determine presence or absence of an aggregate by numerically analyzing a difference waveform of the difference value, wherein
presence or absence of at least two different types of objects included in the image is detected.

2. The inspection device for an aggregate according to claim 1, wherein
the aggregation presence or absence determination unit determines the aggregate by calculating a standard deviation of the difference waveform and comparing the standard deviation with a determination threshold, as numerical analysis of the difference waveform of the difference value.

3. The inspection device for an aggregate according to claim 1, wherein
the aggregation presence or absence determination unit determines the aggregate by calculating a derivative of the difference waveform and comparing the derivative with a determination threshold to perform peak detection, as numerical analysis of the difference waveform of the difference value.

4. The inspection device for an aggregate according to claim 1, wherein
the presence or absence of the foreign matter is detected by calculating a standard deviation of the integrated waveform and comparing the standard deviation with a determination threshold, as numerical analysis of an integrated waveform of the first binarized image.

5. The inspection device for an aggregate according to claim 1, wherein
the presence or absence of the foreign matter is detected by calculating a derivative of the difference waveform and comparing the derivative with a determination threshold to perform peak detection, as numerical analysis of an integrated waveform of the first binarized image.

6. The inspection device for an aggregate according to any one of claims 1 to 5, wherein
the fine particle region detection unit acquires the composite image by a logical product operation of the contour-extracted image and the image, binarizes the composite image, creates the second binarized image, and detects the fine particle region.

7. The inspection device for an aggregate according to any one of claims 1 to 5, further comprising:
an aggregate region determination unit configured to calculate a region of the foreign matter in which the foreign matter is determined to be present and a region including at least the fine particle, specify a region of the fine particle excluding the region of the foreign matter, and calculate a feature of the fine particle included in the region of the fine particle; and
a degree-of-proliferation determination unit configured to determine a degree of proliferation of the fine particle.

8. The inspection device for an aggregate according to claim 7, wherein
the degree-of-proliferation determination unit calculates, as the feature of the fine particle, at least either a total area value of the fine particle or a total number of the fine particles, and the number of the aggregates or an area value of the aggregate, and determines the degree of proliferation of the fine particle.

9. An inspection device for an aggregate for detecting presence or absence of a fine particle aggregate, the inspection device comprising:
an image-capturing unit configured to acquire an image of an inside of a container that holds a liquid containing a fine particle;
a foreign matter presence or absence determination unit configured to binarize the image, create a first binarized image, integrate the first binarized image at least in a vertical or horizontal direction, numerically analyze an integrated value, and detect a foreign matter to determine presence or absence of the foreign matter; and
a re-imaging processing unit configured to change a relative position between the container and the image-capturing unit and acquire an image of the inside of the container again when it is determined that the foreign matter is present.

10. The inspection device for an aggregate according to claim 9, further comprising:
a fine particle region detection unit configured to extract a contour of the image to obtain a contour-extracted image, binarize a composite image of the extracted contour-extracted image and the image, create a second binarized image, and detect a fine particle region;
an integrated signal difference processing unit configured to calculate a difference value between a first integrated signal obtained by integrating the first binarized image and a second integrated signal obtained by integrating the second binarized image; and
an aggregation presence or absence determination unit configured to determine presence or absence of an aggregate by numerically analyzing a difference waveform of the difference value.

11. An inspection method for an aggregate for acquiring an image of an inside of a container that holds a liquid containing a fine particle to detect presence or absence of a fine particle aggregate, the inspection method comprising:
binarizing the image, creating a first binarized image, and determining presence or absence of a foreign matter;
extracting a contour of the image to obtain a contour-extracted image, binarizing a composite image of the extracted contour-extracted image and the image, creating a second binarized image, and detecting a fine particle region;
calculating a difference value between a first integrated signal obtained by integrating the first binarized image and a second integrated signal obtained by integrating the second binarized image; and
determining presence or absence of an aggregate by numerically analyzing a difference waveform of the difference value, wherein
presence or absence of at least two different types of objects included in the image is detected.

12. The inspection method for an aggregate according to claim 11, wherein
the aggregate is determined by calculating a standard deviation of the difference waveform and comparing the standard deviation with a determination threshold, as numerical analysis of the difference waveform of the difference value.

13. The inspection method for an aggregate according to claim 11, wherein
the aggregate is determined by calculating a derivative of the difference waveform and comparing the derivative with a determination threshold to perform peak detection, as numerical analysis of the difference waveform of the difference value.

14. The inspection method for an aggregate according to claim 11, wherein
the presence or absence of the foreign matter is determined by calculating a standard deviation of the integrated waveform and comparing the standard deviation with a determination threshold, as numerical analysis of an integrated waveform of the first binarized image.

15. The inspection method for an aggregate according to claim 11, wherein
the presence or absence of the foreign matter is detected by calculating a derivative of the difference waveform and comparing the derivative with a determination threshold to perform peak detection, as numerical analysis of an integrated waveform of the first binarized image.

16. The inspection method for an aggregate according to any one of claims 11 to 15, wherein
the composite image is acquired by a logical product operation of the contour-extracted image and the image, the composite image is binarized, the second binarized image is created, and the fine particle region is detected.

17. The inspection method for an aggregate according to any one of claims 11 to 15, further comprising:
calculating a region of the foreign matter in which the foreign matter is determined to be present and a region including at least the fine particle, specifying a region of the fine particle excluding the region of the foreign matter, calculating a feature of the fine particle included in the region of the fine particle, and determining a degree of proliferation of the fine particle.

18. The inspection method for an aggregate according to claim 17, wherein
as the feature of the fine particle, at least either a total area value of the fine particle or a total number of the fine particles, and the number of the aggregates or an area value of the aggregate are calculated, and the degree of proliferation of the fine particle is determined.
